# EUROPEAN PATENT SPECIFICATION

(11) **EP 1 493 405 B1**
(45) Date of publication and mention of the grant of the patent: **10.05.2006**
(21) Application number: 04015232.4
(22) Date of filing: 29.06.2004
(51) Int. Cl.: A61F 2/16

(54) **Intraocular lens**
Intraokularlinse
Lentille intraoculaire

(30) Priority: 30.06.2003 JP 2003188628
(43) Date of publication of application: 05.01.2005
(73) Proprietor: Nidek Co., Ltd., Gamagori-shi Aichi-ken 443-0035 (JP)
(72) Inventor: Nagasaka, Shinji, Gamagori-shi Aichi-ken 443-0021 (JP); Sunada, Tsutomu, Toyohashi-shi Aichi-ken 441-3101 (JP)
(74) Representative: Winter, Brandl, Fürniss, Hübner Röss, Kaiser, Polte Partnerschaft Patent- und Rechtsanwaltskanzlei

(56) References cited:
- EP-A- 0 514 263
- US-A- 4 738 680
- US-A1- 2004 034 417
- US-B1- 6 517 577

## Description

### BACKGROUND OF THE INVENTION

### 1. Field of the Invention

The present invention relates to an intraocular lens which is arranged in substitution for a crystalline lens.

### 2. Description of Related Art

Conventionally, there is known an intraocular lens inserted into and arranged inside an eye (inside a crystalline capsule) after an opaque crystalline lens (a stroma of the crystalline lens and cortical fiber cells) caused by a cataract is removed. The intraocular lens includes an optical part which has predetermined refractive power and supporting parts which support the optical part within the eye.

In cataract surgery, a method of surgery using the intraocular lens is a mainstream; however, there is a problem that, after arrangement of the intraocular lens, residual epithelial cells of the crystalline lens in the crystalline capsule propagate towards a posterior capsule side to cause an aftercataract.

The document US6517577B discloses an intraocular lens according to the preamble of claim 1.

### SUMMARY OF THE INVENTION

An object of the invention is to overcome the problems described above and to provide an intraocular lens capable of suppressing an aftercataract with a simple constitution.

To achieve the objects and in accordance with the purpose of the present invention, an intraocular lens includes a foldable optical part which has a shape of refractive surfaces determined in accordance with refractive power to be provided to the optical part, and a supporting part which is jointed to an edge portion of the optical part while being inclined to a plane orthogonal to an optical axis of the optical part, wherein a joint portion of the optical part with the supporting part has a thickness that is deformed due to a stress generated by a pressure applied to the supporting part at the time of arrangement inside an eye regardless of the shape of the refractive surfaces.

Additional objects and advantages of the invention are set forth in the description which follows, are obvious from the description, or may be learned by practicing the invention. The objects and advantages of the invention may be realized and attained by the intraocular lens in the claims.

### BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,
Figs. 1A and 1B are views showing a schematic configuration of an intraocular lens consistent with the preferred embodiment of the present invention;
Fig. 2 is a sectional view showing a state where the intraocular lens is arranged inside an eye;
Figs. 3A and 3B are views illustrating deformation of a joint and its periphery when a stress is produced on a supporting part;
Figs. 4A and 4B are views illustrating measurement of a travel amount of an optical part using a jig; and
Fig. 5 is a graph illustrating the travel amount of the optical part relative to a compression amount of the intraocular lens.

### DETAILED DESCRIPTION OF THE PREFERRED EMBODIMENTS

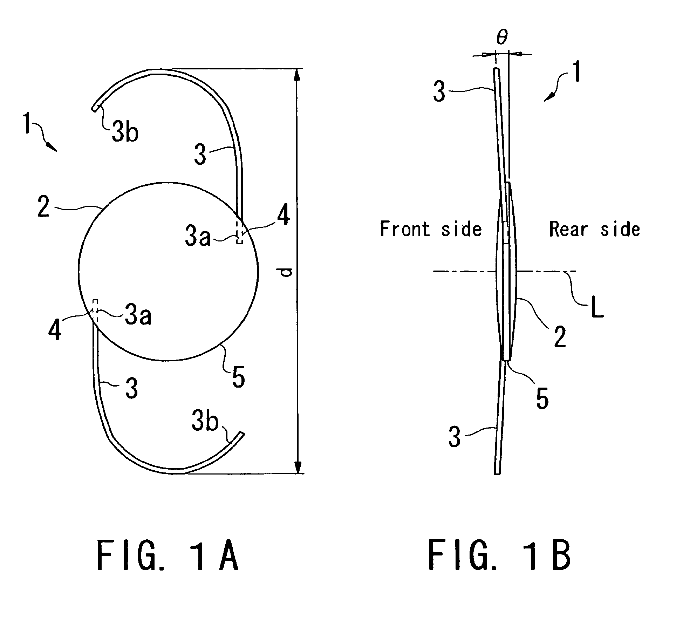

A detailed description of one preferred embodiment of an intraocular lens embodied by the present invention is provided below with reference to the accompanying drawings. Figs. 1A and 1B are views showing a schematic configuration of an intraocular lens consistent with the preferred embodiment of the present invention. Fig. 1A is a front view of the intraocular lens and Fig. 1B is a side view thereof. Besides, in Fig. 1B, a front side refers to a cornea side when the intraocular lens is arranged inside an eye, and a rear side refers to a fundus side. The intraocular lens 1 includes an optical part 2 which has predetermined refractive power, and supporting parts 3 which support and fix the optical part 2 within the eye. The intraocular lens 1 consistent with the preferred embodiment is a three-piece-type one where the optical part 2 and the supporting parts 3 are separately formed and then jointed. Base end portions 3a of the supporting parts 3 are inserted through an edge portion 5 of the optical part 2 to internal predetermined positions, and jointed to the optical part 2 by means of adhesion or welding. Incidentally, joints 4 of the optical part 2 with the supporting parts 3 (hole portions through which the supporting parts 3 (base end portions 3a) are inserted and peripheral portions) are outside an optical region (a region necessary for vision) of the optical part 2.

The optical part 2 is formed of a foldable soft base material for intraocular lenses having a non-hydrous (hydrophobic) or hydrous (hydrophilic) property. The non-hydrous or hydrous soft base material is obtained by blending one or more kinds of monomer which becomes a soft material, and polymerizing and hardening it. Further, it is also obtained by blending a monomer which becomes a hard material as appropriate with the soft material in order to adjust hardness (softness) of the base material to be obtained, and polymerizing and hardening it.

The monomer which becomes the non-hydrous soft material (hereinafter referred to as the "non-hydrous soft monomer") specifically includes acrylate estel such as methyl acrylate, ethyl acrylate, propyl acrylate, 2-ethylhexyl acrylate, n-butyl acrylate, ethylene glycol phenyl ether acrylate, isobutyl acrylate, n-decyl acrylate, cyclohexyl acrylate, and heptyl acrylate.

Also, the monomer which becomes the hydrous soft material (hereinafter referred to as the "hydrous soft monomer") specifically includes: N-vinyllactams such as N-vinylpyrrolidone, α-methylene-N-methylpyrrolidone, and N-vinylcaprolactam; hydroxyl group-bearing (metha)acrylates such as hydroxyethyl(metha)acrylate, hydroxypropyl(metha)acrylate, hydroxybutyl(metha)acrylate, dihydroxypropyl(metha)acrylate, dihydroxybutyl(meta)acrylate, diethylene glycol mono(metha)acrylate, triethylene glycol mono(metha)acrylate, and dipropylene glycol mono(metha)acrylate; (metha)acrylic acids; and (metha)acrylamide, N-methyl(metha)acrylamide, N-ethyl(metha)acrylamide, N-hydroxyethyl(metha)acrylamide, N,N-dimethyl(metha)acrylamide, N,N-diethyl(metha)acrylamide, and N-ethylaminoethyl(metha)acrylamide. Further, polyurethane, silicone and the like are also used as the soft material.

Furthermore, the monomer which becomes the hard material (hereinafter referred to as the "hard monomer") specifically includes methacrylate estel such as methyl methacrylate, ethyl methacrylate, propyl methacrylate, n-butyl methacrylate, isobutyl methacrylate, 2-ethylhexyl methacrylate, 2-methoxyethyl methacrylate, and 2-ethoxyethyl methacrylate.

When the base material is obtained from the soft monomer (non-hydrous or hydrous) or a mixture of the soft monomer and the hard monomer as mentioned above, a cross-linking agent and a polymerization initiator are used as needed. The cross-linking agent specifically includes dimethacrylate estel such as ethylene glycol dimethacrylate, diethylene glycol dimethacrylate and triethylene glycol dimethacrylate, or other material which may be used as the cross-linking agent for forming the base material. The cross-linking agent as above is used within a range of 0.5 to 10 wt% of total weight of the monomer which becomes the base material. In addition, the polymerization initiator specifically includes a material which may be used as the polymerization initiator for forming the base material such as azobis isobutyronitrile, azoisobutyro valeronitrile, benzoin, and methylortho benzoyl benzoate. Further, an ultraviolet absorber such as a benzotriazole series may be added as appropriate to obtain the base material with an ultraviolet absorbing effect. The base material for the optical part 2 has the elastic modulus of preferably from 0.1 MPa to 20 MPa inclusive, more preferably from 0.5 MPa to 5 MPa inclusive.

The optical part 2 is formed by pouring a mixture solvent of the monomer, the cross-linking agent, the polymerization initiator and the like as above into a molding box, and then polymerizing and hardening it. Additionally, the optical part 2 may also be formed by subjecting the polymerized and hardened base material to a cutting process.

Incidentally, a shape of a refractive surface of the optical part 2 in the preferred embodiment is a biconvex shape; however, the it is not limited thereto and may be a shape conventionally employed for the intraocular lens such as a plane-convex shape and a meniscus shape. The shape of the refractive surface of the optical part 2 is determined in accordance with refractive power to be provided to the optical part 2.

Further, a thickness of the joints 4 (edge portion 5) is configured to be a thickness within which the joints 4 are deformed (flexed or bent) with a stress generated by a pressure applied to the supporting parts 3. The thickness for the deformation of the joints 4 is obtained from such conditions as the elastic modulus of the base material used for the optical part 2 and the supporting parts 3, and a thickness (diameter) and a shape of the supporting parts 3. It is preferably a physical thickness of not 0 mm and 0.30 mm or less, more preferably thicker than the supporting parts 3 (base end portions 3a) in the joints 4 and 0.20 mm or less.

On the other hand, the supporting parts 3 are formed of a base material obtained by a material conventionally used for the supporting parts of the intraocular lens. The material specifically includes methyl methacrylate, polypropylene, polyimide and the like. The base material for the supporting parts 3 has the elastic modulus of preferably from 500 MPa to 5000 MPa inclusive, more preferably from 1000 MPa to 3500 MPa inclusive.

Besides, it is essential only that the supporting parts 3 are configured in such a shape that the pressure applied to the supporting parts 3 is transmitted from the base end portions 3a to the joints 4 at the time of arrangement of the intraocular lens 1 inside the eye. In the preferred embodiment, the supporting parts 3 have a shape as shown in Fig. 1A where the thickness is uniform over a whole length, one part in a predetermined length from the base end portion 3a is linear, and the other part therefrom to a tip portion 3b is approximately C-curved. Incidentally, each of the supporting parts 3 in the preferred embodiment has an open-loop shape where one end of the supporting part 3 is jointed with the optical part 2 and the other end is free; however, it is not limited thereto and may have a closed-loop shape which includes no free end.

The supporting parts 3 as above are jointed so as to incline by a predetermined angle θ to the front side as shown in Fig. 1B, for facilitating the deformation of the joints 4 and suppressing protrusion of the optical part 2 to an anterior-segment side of the eye. The joint angle θ of the supporting part 3 (an angle which central axis of the supporting part 3 forms with a plane orthogonal to an optical axis L of the optical part 2) is preferably from 1° to 10° inclusive, more preferably from 3° to 7° inclusive. If the joint angle θ is less than 1°, a desired effect is hardly achieved. On the other hand, if the joint angle θ is more than 10°, a joint strength is decreased. Besides, a general method conventionally used for jointing the optical part 2 and the supporting parts 3 may be used in addition to the adhesion by an adhesive and the welding by heating.

An entire length of the intraocular lens 1 ("d" in Fig. 1A) where the optical part 2 is jointed with the supporting parts 3 is configured to be greater than a diameter of the crystalline lens (crystalline capsule), and apply a predetermined pressure to the supporting parts 3 when the intraocular lens 1 is arranged inside the eye (crystalline capsule).

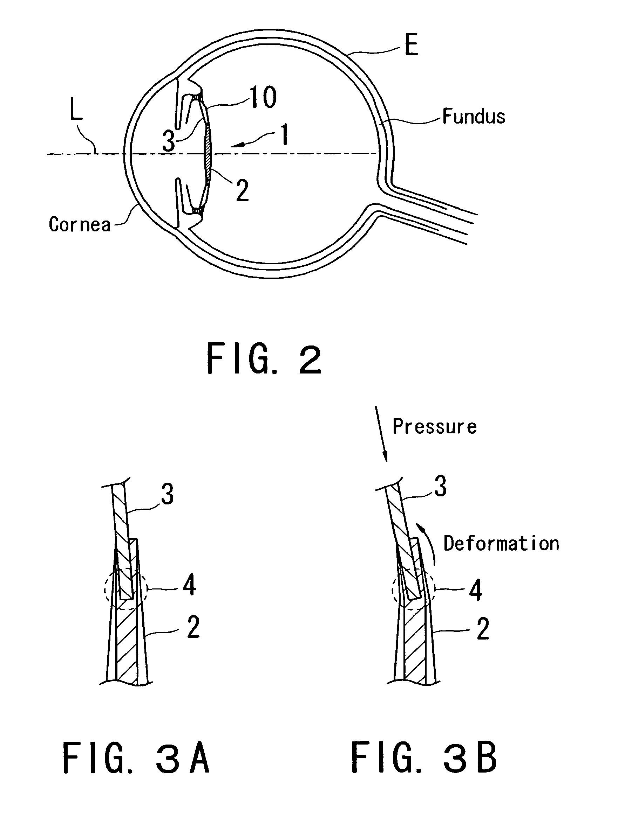

Fig. 2 is a sectional view showing a state where the intraocular lens 1 described above is arranged inside a patient's eye E. The intraocular lens 1, arranged inside the crystalline capsule from which the stroma of the crystalline lens and the like have been removed by known phacoemulsification, is placed in a space smaller than the entire length, so that the supporting parts 3 are applied with the predetermined pressure. Further, the pressure applied to the supporting parts 3 is transmitted to the joints 4 to generate the stress at the joints 4 for deformation. In a state where no pressure is applied to the supporting parts 3 as shown in Fig. 3A, the joints 4 are not deformed. When the pressure is applied to the supporting parts 3 towards the base end portion 3a sides, the stress is generated at the joints 4 for deformation.

Due to the deformation as above, the optical part 2 moves in an optical axis L direction to the fundus side. Thus, the optical part 2 further comes to be adhered to a posterior capsule 10 in such a manner that is pushed against the posterior capsule 10. As a result, a degree of adhesion between the optical part 2 and the posterior capsule 10 is enhanced, so that the aftercataract which is incurred because epithelial cells of the crystalline lens propagate and enter between the optical part 2 and the posterior capsule 10 can be suppressed.

Further, as the optical part 2 is in a state of pushed against the posterior capsule 10, it is prone to influence of a pressure of a vitreous body. Then, when a ciliary muscle tenses to increase the pressure of the vitreous body, the vitreous body pushes the posterior capsule 10 to the cornea side, and the intraocular lens 1 moves in the optical axis direction to the cornea side. On the contrary, when the ciliary muscle relaxes to reduce the pressure of the vitreous body, the intraocular lens 1 moves in the optical axis direction to the fundus side. Thus, as the intraocular lens 1 moves backward and forward in the optical axis direction according to a change in the pressure of the vitreous body due to the tense/relax of the ciliary muscle, the intraocular lens with a single focus may be provided with accommodative power.

Further, as the thickness of the edge portion 5 in the optical part 2 is small, it may fold smaller than the conventional intraocular lens, and an incision provided to the eye E at the time of insertion may be minimized.

### (Example 1)

A mixture composed of 53.95 wt% of ethylene glycol phenyl ether acrylate (EGPEA) and 4.00 wt% of n-butyl acrylate (n-BA) as a non-hydrous soft monomer, 39. 65 wt% of n-butyl methacrylate (n-BMA) as a hard monomer, 2.00 wt% of 1,4-buthane diol diacrylate as a cross-linking agent, and 0.30 wt% of 2-(2'-hydroxy-5'-methacryloxypropyl-3'-tert-butylphenyl)-5-chloro-2H-benzotriazole as an ultraviolet absorber was charged in a reactor, and 0.10 wt% of 2,2-azobisisobutyronitrile as a polymerization initiator was added thereto for initiating a polymerization reaction.

The reactor in which each material was charged was placed in a thermobath kept at 60°C for 24 hours, and then placed in an oven kept at 95°C for 24 hours for proceeding the polymerization reaction. Then, in order to complete the reaction, the reactor was placed in a vacuum oven kept at 95°C for 24 hours to obtain a soft acrylic base material having the elastic modulus of about 1.7 MPa. Next, the obtained soft acrylic base material was subjected to a cutting process at a low temperature to form the optical part 2 having a biconvex shape which is 5.50 mm in diameter and 0.2 mm in thickness for joints 4 (edge portion 5). Incidentally, the optical part 2 was arranged to have desired refractive power of +20D (diopter).

Further, the supporting parts 3 in a shape as shown in Figs. 1A and 1B were formed using a wire rod made of polymethyl methacrylate (with the elastic modulus of 3000 MPa). The supporting parts 3 were 0.15 mm in thickness.

Furthermore, two holes were formed for inserting the supporting parts 3 into the optical part 2, and the base end portions 3a of the supporting part 3 were inserted therein and welded by heating to form the intraocular lens 1 of 12.5 mm in entire length. Besides, the joints 4 are parts which are out of a range of 4.6 mm in diameter with reference to the center of the optical part 2 to be outside an optical region of the optical part 2. Further, the joint angle θ of the supporting parts 3 with the optical part 2 was 3°.

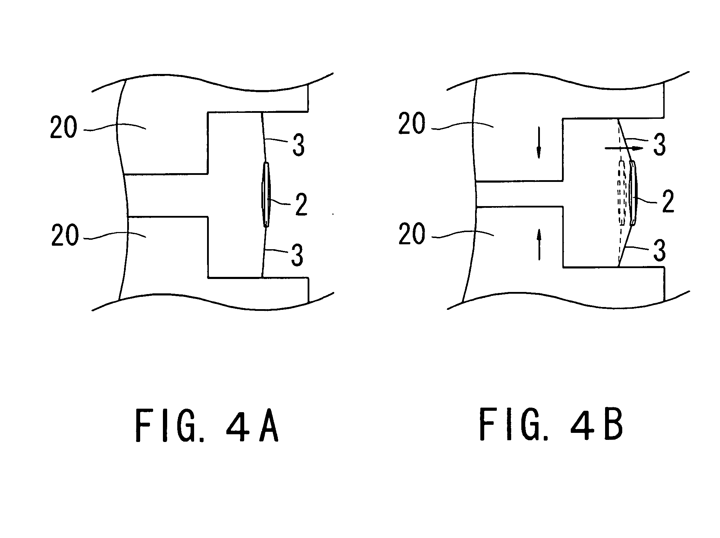

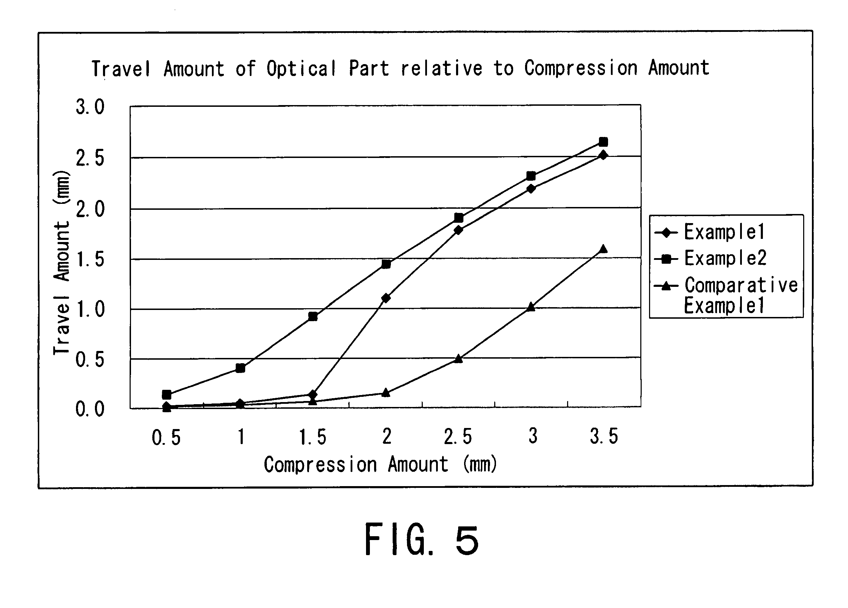

Next, the obtained intraocular lens 1 was set in a jig 20 as shown in Fig. 4A, and an opening diameter of the jig 20 was reduced stepwise to generate a stress (compressive load) at the intraocular lens 1, and a travel amount of the optical part 2 at that time was measured. Incidentally, the opening diameter of the jig 20 in the beginning of the measurement was 12.5 mm, and reduced by 0.5 mm steps thereafter. Results are shown in Fig. 5. Besides, during the measurement, the joints 4 were observed through a microscope. In the intraocular lens in Example 1, deformation of the joints 4 in accordance with the reduction in the opening diameter of the jig 20 was observed.

### (Example 2)

The intraocular lens 1 was obtained under the same conditions as in Example 1 except that the joint angle θ of the supporting parts 3 was 7°. Then, a travel amount of the optical part 2 was measured using the jig 20 similarly to Example 1. Results are shown in Fig. 5. In the intraocular lens in Example 2, similarly to the intraocular lens in Example 1, deformation of the joints 4 in accordance with the reduction in the opening diameter of the jig 20 was observed.

### (Comparative Example 1)

As an example of a conventional intraocular lens in which the joints 4 do not deform due to the stress, the intraocular lens 1 was obtained under the same conditions as in Example 1 except that the thickness for the joints 4 (edge portion 5) was 0.35 mm. Then, a travel amount of the optical part 2 was measured using the jig 20 similarly to Example 1. Results are shown in Fig. 5. In the intraocular lens in Comparative Example 1, different from the intraocular lens in Example 1, deformation of the joints 4 in accordance with the reduction in the opening diameter of the jig 20 was not observed.

### (Results)

As shown in Fig. 5, in the intraocular lenses in Examples 1 and 2, the travel amount of the optical part 2 in the optical axis direction exceeded 1.0 mm when the compression amount was 2 mm. As a diameter of the crystalline capsule where the intraocular lens is actually arranged is about 9.0 mm to 10.0 mm, when the intraocular lens of about 12.5 mm in entire length in the preferred embodiment is used, it adheres to the posterior capsule stronger than the conventional intraocular lens (Comparative Example 1).

As described above, the intraocular lens consistent with the present invention is insertable into the eye from a small incision, and capable of suppressing the aftercataract. Further, the travel amount of the optical part in the optical axis direction according to the tense/relax of the ciliary muscle is so large that the accommodative power may be provided.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1. An intraocular lens comprising:
a foldable optical part (2) which has a shape of refractive surfaces determined in accordance with refractive power to be provided to the optical part; and
a supporting part (3) which is jointed to an edge portion (5) of the optical part while being inclined to a plane orthogonal to an optical axis (L) of the optical part,
**characterized in that**
a joint portion (4) of the optical part with the supporting part has a thickness that is deformed due to a stress generated by a pressure applied to the supporting part at the time of arrangement inside an eye, regardless of the shape of the refractive surfaces.

2. The intraocular lens according to claim 1, wherein the supporting part is formed of a base material with higher elastic modulus than elastic modulus of a base material forming the optical part.

3. The intraocular lens according to claim 2,
wherein the optical part is formed of the base material with the elastic modulus of from 0.1 MPa to 20 MPa inclusive, more preferably from 0.5 MPa to 5 MPa inclusive, and
the supporting part is formed of the base material with the elastic modulus of from 500 MPa to 5000 MPa inclusive, more preferably from 1000 MPa to 3500 MPa inclusive.

4. The intraocular lens according to one of claims 1 to 3, wherein the joint portion of the optical part with the supporting part has a physical thickness, and has a thickness of 0.3 mm or less, more preferably 0.2 mm or less.

5. The intraocular lens according to one of claims 1 to 4, wherein the supporting part is jointed to the edge portion of the optical part at an inclined angle of from 1° to 10° inclusive, more preferably from 3° to 7° inclusive.

6. The intraocular lens according to one of claims 1 to 5, wherein the optical part has the shape of the refractive surfaces which is one of a biconvex shape, a plane-convex shape and a meniscus shape.

## Patentansprüche

1. Intraokularlinse, umfassend:
ein faltbares optisches Teil (2), das die Form von Brechungsoberflächen aufweist, wobei diese Form so festgelegt ist, dass sie dem optischen Teil die gewünschte Brechkraft zur Verfügung stellt; und
ein tragendes Teil (3), das mit einem Randabschnitt (5) des optischen Teils verbunden ist, wobei es zu einer Ebene geneigt ist, die senkrecht auf der optischen Achse (L) des optischen Teils steht,
**dadurch gekennzeichnet, dass**
ein Verbindungsabschnitt (4) des optischen Teils mit dem tragenden Teil eine Dicke aufweist, die, ungeachtet der Form der Brechungsoberflächen, durch eine Spannung, die durch einen Druck, der zum Zeitpunkt des Anordnens innerhalb des Auges auf das tragende Teil ausgeübt wird, verformt wird.

2. Intraokularlinse nach Anspruch 1, wobei das tragende Teil aus einem Grundmaterial mit einem höheren Elastizitätsmodul als dem Elastizitätsmodul eines Grundmaterials, das das optische Teil bildet, gebildet wird.

3. Intraokularlinse nach Anspruch 2, wobei das optische Teil aus dem Grundmaterial mit dem Elastizitätsmodul von 0,1 MPa bis einschließlich 20 MPa gebildet wird, mehr bevorzugt von 0,5 MPa bis einschließlich 5 MPa, und das tragende Teil aus dem Grundmaterial mit dem Elastizitätsmodul von 500 MPa bis einschließlich 5000 MPa, mehr bevorzugt von 1000 MPa bis einschließlich 3500 MPa, gebildet wird.

4. Intraokularlinse nach einem der Ansprüche 1 bis 3, wobei der Verbindungsabschnitt des optischen Teils mit dem tragenden Teil eine physikalische Dicke aufweist, und eine Dicke von 0,3 mm oder weniger, mehr bevorzugt von 0,2 mm oder weniger, aufweist.

5. Intraokularlinse nach einem der Ansprüche 1 bis 4, wobei das tragende Teil mit dem Randbereich des optischen Teils in einem geneigten Winkel von 1°C bis einschließlich 10°C, mehr bevorzugt in einem Winkel von 3°C bis einschließlich 7°C, verbunden ist.

6. Intraokularlinse nach einem der Ansprüche 1 bis 5, wobei das optische Teil die Form der Brechungsoberflächen aufweist, nämlich entweder eine bikonvexe Form, eine plankonvexe Form oder eine Meniskusform.

## Revendications

1. Lentille intra-oculaire comprenant :
une pièce optique pliable (2) qui a la forme des surfaces de réfraction déterminée conformément à la puissance de réfraction à fournir à la pièce optique ; et
une pièce de support (3) qui est assemblée sur une partie formant bord (5) de la pièce optique tout en étant inclinée par rapport à un plan orthogonal par rapport à un axe optique (L) de la pièce optique,
**caractérisée en ce que**
une partie d'assemblage (4) de la pièce optique avec la partie de support a une épaisseur qui est déformée en raison d'une contrainte générée par une pression appliquée sur la pièce de support au moment de la mise en place dans un oeil, quelle que soit la forme des surfaces de réfraction.

2. Lentille intra-oculaire selon la revendication 1, dans laquelle la pièce de support est formée à partir d'un matériau de base ayant un module d'élasticité supérieur au module d'élasticité d'un matériau de base formant la pièce optique.

3. Lentille intra-oculaire selon la revendication 2, dans laquelle la pièce optique est formée à partir d'un matériau de base avec un module d'élasticité de 0,1 MPa à 20 MPa inclus, de préférence de 0,5 MPa à 5 MPa inclus, et
la pièce de support est formée à partir d'un matériau de base ayant un module d'élasticité de 500 MPa à 5000 MPa inclus, de préférence de 1000 MPa à 3500 MPa inclus.

4. Lentille intra-oculaire selon l'une quelconque des revendications 1 à 3, dans laquelle la partie d'assemblage de la pièce optique avec la pièce de support a une épaisseur physique, et a une épaisseur de 0,3 mm ou moins, de préférence de 0,2 mm ou moins.

5. Lentille intra-oculaire selon l'une quelconque des revendications 1 à 4, dans laquelle la pièce de support est assemblée sur la partie de bord de la pièce optique selon un angle incliné de 1° à 10° inclus, de préférence de 3° à 7° inclus.

6. Lentille intra-oculaire selon l'une quelconque des revendications 1 à 5, dans laquelle la pièce optique a la forme des surfaces de réfraction qui est l'une parmi une forme biconvexe, une forme plano-convexe et une forme de ménisque.
